# EUROPEAN PATENT APPLICATION

(11) **EP 4 148 104 A1**
(43) Date of publication of application: **15.03.2023**
(21) Application number: 22194338.4
(22) Date of filing: 07.09.2022
(51) Int. Cl.: C10G 2/00, C25B 1/04

(54) **A METHOD OF PRODUCING CLIMATE-NEUTRAL FUEL, AND A SYSTEM FOR CARRYING OUT SAID METHOD**

(30) Priority: 09.09.2021 HU 2100318
(71) Applicant: Mifler Consulting Kft., 1026 Budapest (HU)
(72) Inventor: Mezei, Ferenc, 1026 Budapest (HU); Mezei, Rita, 1026 Budapest (HU); Papp, Géza Bálint, 2030 Érd (HU)
(74) Representative: Kacsuk, Zsófia

(57) **Abstract**

The invention relates to a method of producing climate-neutral fuel, in which producing electrical energy using solar cells (10) and with the energy produced:
(a) producing carbon dioxide and hydrogen from air and/or water,
(b) preparing a hydrocarbon mixture from carbon dioxide and hydrogen gases using a one or more step chemical and/or electrochemical process,
(c) separating the hydrocarbon mixture into one or more fuel fractions (5a) and one or more energy carrier fractions (5b),
(d) producing climate-neutral fuel from the fuel fraction (5a),
(e) producing energy using the one or more energy carrier fractions (5b), while steps (a)-d) are repeated and at least part of the instantaneous energy demand of steps (a)-d) is covered by the energy thus produced.

The invention further relates to a system (100) for carrying out the method according to the invention.

## Description

The present invention relates to a method of producing climate-neutral fuel.

The present invention also relates to a system for carrying out the method according to the invention.

The use of oil-based fuels for transportation is one of the largest sources of climate catastrophic carbon dioxide (CO2) emissions into the air. In principle, this harmful side effect of transport could be eliminated by switching to electric motors if large-scale electricity generation were carbon-free, i.e. using only nuclear or renewable energy. Unfortunately, we are a long way from this in the global context of electricity production. On the other hand, the large-scale use of batteries has its own environmental problems due to battery production and waste management.

It would therefore be much simpler to use hydrocarbon fuels (hereinafter: fuels) with conventional properties, for the production of which we do not use fossil coal or hydrocarbons, either as a constituent of the fuel or as a source of energy for the processes needed to produce them. This would have the particular advantage of avoiding the need to replace the existing vehicle fleet with electric propulsion, which would take decades and be very costly, especially in aviation.

Since the beginning of the Second World War, large-scale production of synthetic fuels has been a regular practice in cases where oil is not directly available, only coal, e.g. for political reasons (war, embargo, etc.) or where there is a lot of natural gas instead. The so-called Fischer-Tropsch process is a catalysed chemical reaction in which a synthesis gas containing a mixture of carbon monoxide and hydrogen is converted into a hydrocarbon mixture containing carbon chains of different lengths. During the process "artificial crude oil" is produced, which can then be broken down into hydrocarbon components (so-called fractions or distillates) with different carbon chains using processes common in oil refining (e.g. fractional distillation) to produce synthetic petrol, synthetic kerosene or synthetic gas oil and many other compounds. The disadvantage of the above method is that the use of synthetic fuels is as harmful to the climate as natural petroleum derivatives, since the carbon monoxide used is of fossil origin, e.g. produced from coal or natural gas. Hydrogen can also be produced using e.g. natural gas and fossil energy.

Recently, it has been recognised that it is possible to produce the hydrogen gas and carbon monoxide gas needed to produce synthetic fuels not from fossil fuels, but simply from water and/or air using electricity. The process involves extracting hydrogen from water and carbon monoxide from the carbon dioxide content of air or natural waters. The burning of such fuel returns to the air exactly the amount of carbon dioxide that was originally extracted for production. So, burning these fuels does not increase the carbon dioxide content of the air, i.e. it is climate neutral. This requires that, in addition to the raw materials, the energy used in the process (electricity and heat) is also produced in a 100% carbon emission free way. The latter can be achieved by using renewable energy sources, including solar energy.

We recognised that the production of climate-neutral hydrocarbon fuels could benefit from the use of solar energy, as it is a mature, climate-neutral energy production technology that can be applied in many parts of the world.

We also recognised that there is currently no climate-neutral fuel production method that can operate reliably and efficiently without an external fossil fuel source (e.g. grid electricity) with the available solar energy production which constantly changes during the day, with the weather and with the seasons. It is not possible with the currently known solutions to ensure that sufficient and fully climate-neutral electricity is always available for all functional and safety tasks required for safe operation and fuel production, even during production breaks of the solar panels.

We recognised that the production of climate-neutral fuels generates significant quantities of hydrocarbon fractions - also climate-neutral - which can either not be sold or can only be sold at a low price. We recognised that these by-product hydrocarbons, just like a part of the primary produced hydrocarbon fuels, have an energy storage function, i.e. they can be used to power electrical generators at any time, and the energy stored in them can be converted back into electricity or heat energy in a climate-neutral way, with an efficiency corresponding to the efficiency of the thermal power plant used. In other words, the usable electrical energy production of on-site solar panels is made independent or less dependent on the time of day and weather by storing part of the electrical energy produced in the form of hydrocarbons as by-products for the time when the output of the solar panels needs to be replaced or supplemented to provide a certain level of continuous electrical energy supply. If necessary, part of the hydrocarbon fuel produced can also be converted back into electricity, for example in order to ensure the power supply in the event of a power outage due to the failure of the solar panels, in particular to meet the energy needs of the safety equipment. In this way, the fuel production plant could be made independent of the external electricity grid, which would not necessarily provide climate-neutral electricity. The plant can therefore be made independent from external energy suppliers through the energy stored in the fuel and by-products produced and can be operated in a fully climate-neutral way.

We also recognised that burning the hydrocarbons produced as by-products can not only compensate for the reduced energy production of solar panels, but can also be used to increase the share of the desired fuel in the global production process. Overall, the efficiency of producing the desired type of fuel (e.g. kerosene) can be increased, in a climate-neutral way.

The invention aims to provide a method and system that is free from the disadvantages of the prior art solutions.

The aim of the invention is in particular to provide a method and system for making the available electrical energy production of solar cells independent or less dependent on the time of day and weather by storing part of the electrical energy produced as by-product hydrocarbons. This not only ensures the autonomy and reliability of the method and system for fuel production, but also increases the efficiency of the desired fuel production, all in a fully climate-neutral way.

The problem according to the invention has been solved by a method of producing climate-neutral fuel according to claim 1, and by a system according to claim 8.

The essence of the invention is to use solar panels to generate electricity, and the method uses the solar energy generated to produce the desired fuel in several steps and inevitably by-products. By way of example, the detailed process is illustrated for the Fischer-Tropsch process in one preferred embodiment:
(a) producing carbon dioxide and hydrogen from air and/or water,
(b) preparing a hydrocarbon mixture from carbon dioxide and hydrogen gases using a one or more step chemical and/or electrochemical process,
(c) separating the hydrocarbon mixture into one or more fuel fractions and one or more energy carrier fractions,
(d) producing climate-neutral fuel from the fuel fraction,
(e) producing energy using the one or more energy carrier fractions, while steps a)-d) are repeated and at least part of the instantaneous energy demand of steps a)-d) is covered by the energy thus produced.

Some preferred embodiments of the invention are defined in the dependent claims.

Further details of the invention are described by means of exemplary embodiments and drawings. In the drawings
Figure 1 is a schematic perspective view showing the main elements involved in an exemplary method according to the invention;
Figure 2 is a schematic view of an exemplary carbon dioxide production unit of a system according to the invention;
Figure 3 is a schematic view of an exemplary hydrogen production unit of a system according to the invention;
Figure 4 is a schematic view of an exemplary fractionation unit of a system according to the invention.

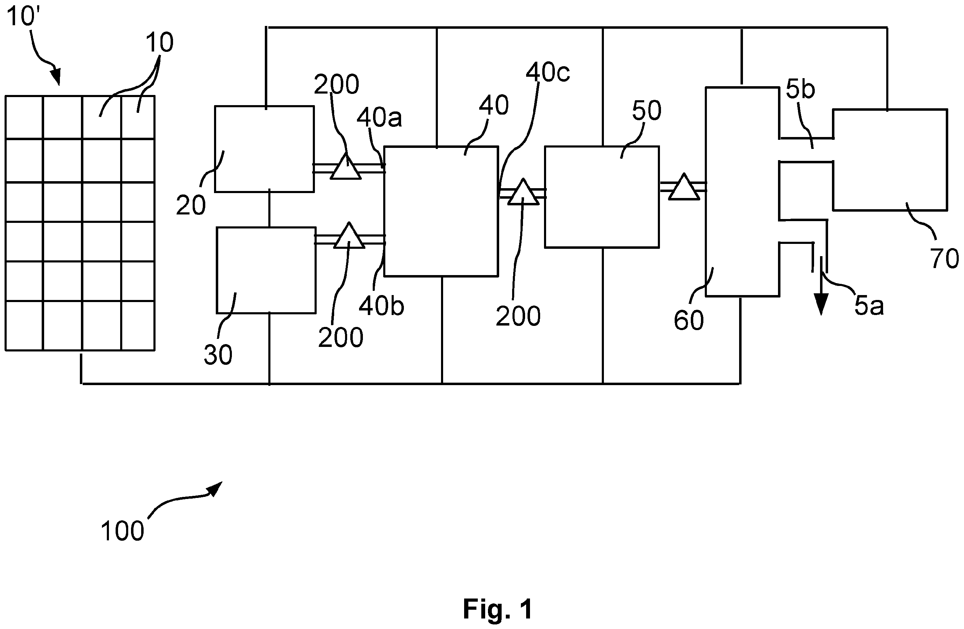

Figure 1 shows a schematic, exemplary perspective view of the main elements involved in a method of producing climate-neutral fuel according to the invention. It is noted that, although the steps of the method are presented sequentially in the following, since the method according to the invention is a time-spanning process, each step can be performed in parallel as appropriate. In the context of the present invention, climate neutrality is understood to mean that the hydrocarbon fuel is produced without carbon dioxide emissions, i.e. neither the production of feedstock nor the production of the energy for the production method substantially increases the carbon dioxide content of the atmosphere.

To implement the method according to the invention, solar panels 10 are used to generate electricity in a way known per se. The solar panels 10 can be preferably arranged into a solar panel park, as will be apparent to the person skilled in the art. For industrial scale fuel production, the combined peak power of the solar panels 10 is preferably of the order of 100 MW, which can be achieved by a solar panel park of a few km² area.

In step a) of the method, the electricity produced by the solar panels 10 is used to produce carbon dioxide and hydrogen from air and/or water. The hydrogen is produced from water by a known method, such as electrolysis, as is known to the person skilled in the art. The source of the water may be, for example, a river, sea, lake, groundwater, etc. Prior to electrolysis, the water may be treated in the usual way, e.g. purified or added. Since the air in the atmosphere virtually always contains all water vapour, in a particularly preferred embodiment the water required for the production of hydrogen is extracted from the atmospheric air, e.g. by condensing the humidity of the air. The advantage of this is that the method according to the invention can be carried out in geographical locations where the necessary amount of water is not otherwise available.

The carbon dioxide required for the process of the invention is preferably produced by extracting carbon dioxide from the air, for example using carbon dioxide traps from a company called ClimeWorks. The carbon dioxide in the air passing through the trap is collected by special filters, from which the carbon dioxide can then be extracted in concentrated form. Only electricity is required to operate the traps. In another possible embodiment, the carbon dioxide is extracted from water that is in solution equilibrium with air in terms of carbon dioxide content, i.e. natural waters (e.g. seawater). One possible example is the so-called Ocean Thermal Energy Conversion (OTEC) process, where 2-3% of the carbon dioxide content of seawater can be extracted.

In the next step (b) of the method according to the invention, hydrocarbons are produced from hydrogen and carbon dioxide by a one or more step chemical and/or electrochemical process.

In the case of a preferred embodiment, synthesis gas is created using the produced hydrogen and carbon dioxide. Synthesis gas is a mixture of hydrogen and carbon monoxide in a certain proportion, e.g. this is the starting material for the Fischer-Tropsch process. In the context of the present invention, synthesis gas can be created as a mixture of carbon monoxide produced from carbon dioxide and hydrogen. It is noted that in the context of the present invention, the term "gas" includes both gaseous and liquid gas. A hydrocarbon mixture is prepared from the synthesis gas using a chemical process, preferably for example the Fischer-Tropsch process. In this process, the carbon and hydrogen atoms of the synthesis gas are separated in the presence of a catalyst and the carbon atoms are organized into chains of different lengths, while the hydrogen atoms are attached to them. The process results in a hydrocarbon mixture (synthetic oil) similar to natural oil, which is a complex mixture of different hydrocarbon compounds.

In the next step c) of the method, the hydrocarbon mixture, which contains hydrocarbon compounds with different carbon chain lengths and structures, is not separated individually, but in groups (so-called fractions) according to their application, in a known way, e.g. by fractional distillation. In this process, the hydrocarbon mixture is heated to several hundred °C and fed into the vaporisation space of a distillation tower containing several levels (trays), known per se. Here the liquid and vapour components separate. The lower boiling point components flow upwards as vapour, the higher boiling point components flow downwards as liquid. The vapours traveling upwards gradually cool down, so that the different fractions precipitate on the different trays. The components with the highest boiling points at the bottom and the lowest boiling points at the top precipitate and become liquid. In descending order of temperature, the different fractions are: crude diesel, kerosene, heavy gasoline, crude gasoline. In the method according to the invention, the hydrocarbon mixture is separated into one or more fuel fractions 5a and one or more energy carrier fractions 5b. In the context of the present invention, the fuel fraction 5a is understood to be a hydrocarbon group corresponding to a particular type of fuel (e.g. kerosene) to be produced by the method of the invention, falling within a particular carbon chain length range (e.g. C11-C12 for kerosene), as is known to the person skilled in petrochemistry. In the context of the present description, the one or more energy carrier fractions 5b is understood to be the residue of the hydrocarbon mixture above the fuel fraction 5a, i.e. the other fractions resulting from the fractionation process.

In the next step d) of the method, the fuel fraction 5a is used to produce a climate-neutral fuel or fuels. In this process, the fuel fraction 5a is purified or refined, as appropriate, by methods known and customary in petroleum refining. In a preferred embodiment, kerosene is produced as a climate neutral fuel, i.e. the fuel fraction 5a is the hydrocarbon group corresponding to kerosene.

In the next step (e) of the method according to the invention, energy is produced using one or more of the energy carrier fractions 5b, while the above steps for producing the fuel are repeated and at least a part of the instantaneous energy demand of the steps is covered by the energy thus produced. In the context of the present invention, the instantaneous energy requirement of the steps is understood to be the total energy required for the production process at any given time. In the event of an excessive reduction or termination of energy production of the solar panels 10, the lost electrical power can be replaced by the energy produced in step e). It is noted that since the amount of energy that can be generated in step e) depends on the limited amount of available energy carrier fractions 5b, in the event of an expected prolonged loss of power production from the solar panels 10, the power produced in step e) will also be used to safely complete steps a) to d), i.e. to safely shut down the entire fuel production process.

Energy production using fuel fractions 5b can be achieved by methods known per se, e.g. using a thermal power plant by burning fuel fractions 5b in combustion engines or boilers. Preferably at least part of the thermal energy so produced is converted into electrical energy by means of generators. Since both electrical and thermal energy are required for the steps of the method according to the invention, the energy production by combustion of the energy carrier fractions 5b is highly efficient, since the generated waste heat can be used to heat the hydrocarbon mixture, for example in the fractionation step. It is noted that the energy carrier fractions 5b can be "burnt" in catalysed or non-catalysed, equilibrium or non-equilibrium chemical processes, as appropriate, and thus generate thermal energy and, if required, electrical energy, as is known to the skilled person. By burning the otherwise by-product energy carrier fractions 5b and feeding the energy produced back into the process, the amount of fuel produced and the efficiency of the production can be increased. On the other hand, energy can be stored in the form of the energy carrier fractions 5b in case the solar panels 10 are not able to produce sufficient energy to cover the energy demand of the method steps, including the need for maintenance and operational safety during breaks in fuel production, for example due to bad weather. In other words, the lost capacity of the solar panels 10 can be partially or even fully replaced by converting the energy carrier fractions 5b into energy. In a preferred embodiment, more than half, or in some cases all, of the instantaneous energy requirements of the fuel production steps are met by energy produced using one or more of the energy carrier fractions 5b.

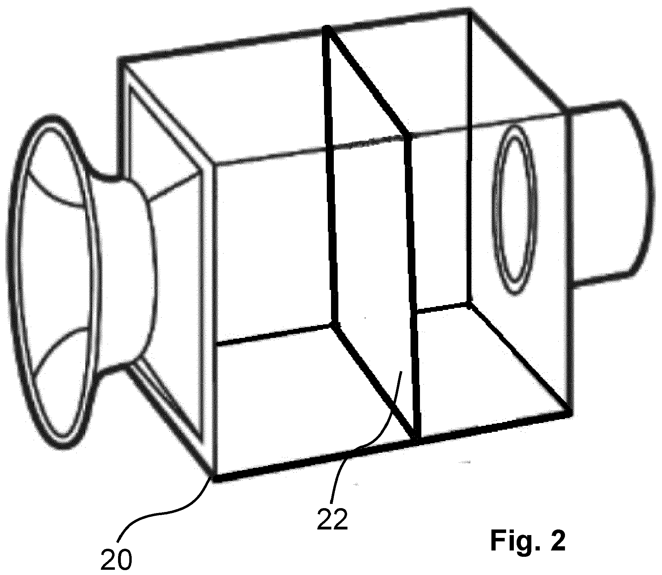

The present invention also relates to a system 100 for carrying out the method according to the invention. A schematic view of the main elements of the system 100 is shown in Figure 1. The system 100 is based on solar panels 10 that generate electrical energy as a function of the irradiation from the sun. The solar panels 10 can preferably be arranged in a solar panel park 10'. The maximum power of the solar panel park 10' can be easily scaled by the number of solar panels 10, so that several MW of electrical power can be generated if required. The system 100 comprises a carbon dioxide production unit 20 for extracting carbon dioxide from the environment and a hydrogen production unit 30 for producing hydrogen from water, connected to the solar farm 10'. Figure 2 shows an exemplary embodiment of the carbon dioxide production unit 20, configured as a carbon dioxide trap for extracting carbon dioxide from the air. As the carbon dioxide flows through filters 22 of the unit 20, it is "trapped" and collected as the filtered air passes through unobstructed. When the 22 filters are heated, the carbon dioxide is released again and is collected and discharged. The air can be continuously circulated using electrical energy, and the filter 22 can be heated using Joule heat or a hot medium (e.g. water) circulated in a heat exchanger. Note that carbon dioxide can also be extracted from natural waters (e.g. the sea), as mentioned earlier.

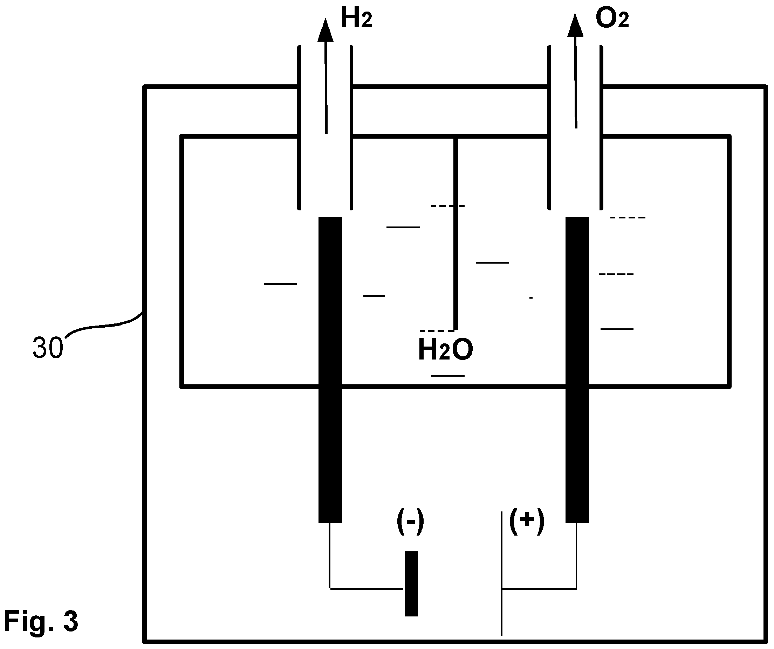

Figure 3 shows an exemplary embodiment of a hydrogen production unit 30. In this case, electrodes are placed in a tank full of water and an electrical voltage is applied to them. The hydrogen is extracted at the cathode and drained from the tank. Note that although in Figure 1 the system 100 has only one unit 20 and one unit 30, of course the system 100 can contain several units 20, 30.

The system 100 includes a unit 40 connected to units 20, 30, for example, to produce synthesis gas from carbon dioxide and hydrogen. The conversion of carbon dioxide to carbon monoxide can be done by the unit 40 or by a separate device inserted between the units 20 and 40 (not shown). The hydrogen and carbon dioxide (or carbon monoxide) enter a tank through inlets 40a, 40b of the unit 40 and exit as a synthesis gas through an outlet 40c of the unit 40. The system 100 also includes a conversion unit 50, connected to the outlet 40c of the unit 40, for producing a hydrocarbon mixture from the synthesis gas. Unit 50 may be configured as a rigid-walled vessel with catalyst plates, in which, under the appropriate pressure (some to tens of atmospheres) and temperature (typically 150-300 degrees Celsius), the synthesis gas is converted to a hydrocarbon mixture during Fischer-Tropsch synthesis.

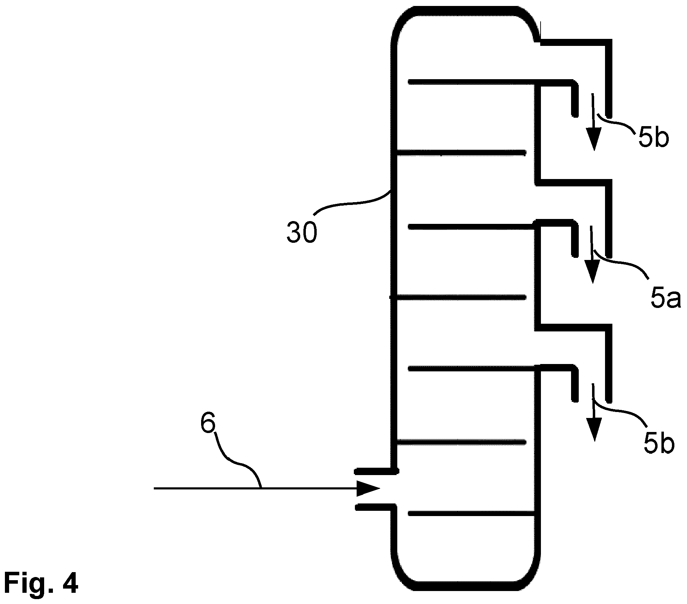

The system 100 includes a fractionation unit 60 for separating the hydrocarbon mixture into a fuel fraction 5a and one or more energy carrier fractions 5b. Figure 4 shows an exemplary embodiment of a unit 60 according to the invention, wherein the unit 60 is configured as a distillation tower. The hydrocarbon mixture produced by the unit 50 is introduced into the unit 60, but prior to this, the hydrocarbon mixture is heated to a temperature of a few hundred degrees Celsius, for example, by means of a tube furnace (not shown). The heating of the hydrocarbon mixture can be done, for example, by electrical energy (Joule heat) or by a heat exchanger, as is obvious to the skilled person. In the unit 60, the hydrocarbon mixture is separated into one or more fuel fractions 5a and one or more energy carrier fractions 5b, as described above. The one or more fuel fractions 5a are used to make a climate neutral fuel, and the one or more energy carrier fractions 5b are used for the further step of the method according to the invention. The energy carrier fractions 5b may, of course, be stored (e.g. in tanks), as appropriate, prior to their use. It is noted that the carbon dioxide, carbon monoxide, hydrogen and synthesis gas may be transported, preferably via pipelines, by means of pumps 200 between the respective units 20, 30, 40, 50, 60.

The system 100 also comprises a thermal power plant 70 connected to units 20, 30, 40, 50, 60 units capable of utilising the one or more energy carrier fractions 5b. In the context of the present invention, the thermal power plant 70 is understood to be an energy conversion device capable of generating thermal energy by combustion of one or more energy carrier fractions 5b and electrical energy therefrom, and transmitting it to the units 20, 30, 40, 50, 60, as required. The thermal power plant 70 preferably comprises the usual components, such as an apparatus (e.g., a steam boiler, an combustion engine) for burning one or more energy carrier fractions 5b, an electric generator and other usual elements, such as a turbine (not indicated in the figures), as known to the skilled person.

In a preferred embodiment, the solar panel park 10' is sized to meet the combined instantaneous power demand of units 20, 30, 40, 50, 60, i.e., to provide sufficient electrical power to operate the units 20, 30, 40, 50, 60 at full capacity under sufficient irradiance. That is, in the normal case, under appropriate seasonal and weather conditions, the entire energy demand of the units 20, 30, 40, 50, 60 is met by the solar panel park 10' and the climate neutral fuel is produced directly from solar energy. However, as the irradiance decreases, the energy production of the solar panel park 10' is also reduced or even completely ceased (e.g. at night). In the method according to the invention, the lost energy production of the solar panel park 10' is partially or completely covered by the thermal power plant 70 by burning one or more of the energy carrier fractions 5b.

The thermal power plant 70 is sized to meet the combined instantaneous energy demand of the units 20, 30, 40, 50, 60 in such a way that by temporarily replacing the solar panel park 10', it is capable of operating the units 20, 30, 40, 50, 60 at full capacity on their own. Some of the energy produced by the thermal power plant 70 is electricity and some is thermal energy. The operation of the units 20, 30, 40, 50, 60 requires partly electrical energy and partly thermal energy (e.g. heating of hydrocarbon mixtures). For the latter, the thermal energy generated in the thermal power plant 70 is directly transferred, e.g. by means of a heat exchanger, to the respective units 20, 30, 40, 50, 60.

The method and system 100 of the invention can produce commercial quantities of climate-neutral fuel (e.g. kerosene) with essentially no utility connection and no feedstock supply. Everything comes from the sun and the air, and apart from component wear and tear, operation is autonomous. Due to the enormous mass and constant movement and mixing of the atmosphere, there will be no local carbon dioxide concentration changes. In the case of hydrocarbon fuel production, it is a cycle: during use, the fuel returns to the air as water and carbon dioxide.

Hydrocarbons can also be produced by other known chemical and/or electrochemical processes using carbon dioxide and hydrogen. For example, a mixture of hydrogen and carbon dioxide gases is used to produce alcohols by a known chemical process, from which hydrocarbons can be produced by a further chemical process. Such chemical and/or electrochemical processes are well known to the person skilled in the art, and the above-described method can be readily adapted to the case where hydrocarbons are produced by such processes.

It is clear that alternative embodiments to those disclosed herein may be envisaged by the practitioner, but within the scope of protection defined by the claims.

## Claims

1. A method of producing climate-neutral fuel, **characterised by** producing electrical energy using solar cells (10) and with the energy produced:
(a) producing carbon dioxide and hydrogen from air and/or water,
(b) preparing a hydrocarbon mixture from carbon dioxide and hydrogen gases using a one or more step chemical and/or electrochemical process,
(c) separating the hydrocarbon mixture into one or more fuel fractions (5a) and one or more energy carrier fractions (5b),
(d) producing climate-neutral fuel from the fuel fraction (5a),
(e) producing energy using the one or more energy carrier fractions (5b), while steps a)-d) are repeated and covering at least part of the instantaneous energy demand of steps a)-d) by the produced energy.

2. The method according to claim 1, **characterized by** in step b) producing a synthesis gas in the form of a mixture of carbon monoxide and hydrogen from carbon dioxide and hydrogen, and then producing a hydrocarbon mixture from the synthesis gas using a chemical process, preferably a Fischer-Tropsch process.

3. The method according to claim 1 or 2, **characterized by** more than half of the instantaneous energy demand of steps a)-d) is covered by the energy produced using the one or more energy carrier fractions (5b).

4. The method according to any one of claims 1 to 3, **characterised in that** the energy produced in step e) is thermal energy obtained by combustion of one or more of the energy carrier fractions (5b).

5. The method according to claim 4, **characterized by** converting at least a portion of the thermal energy into electrical energy.

6. The method according to any one of claims 1 to 5, **characterized by** producing kerosene as a climate-neutral fuel in step d).

7. The method according to any one of claims 1 to 6, **characterized by** producing carbon dioxide in step a) by extracting carbon dioxide from the air and hydrogen by electrolysis using water from the humidity in the air.

8. The method according to any one of claims 1 to 7, **characterized by** performing the separation of the hydrocarbon mixture into fractions in step c) by fractional distillation.

9. The method according to any one of claims 1 to 8, **characterised by** storing a portion of the climate-neutral fuel, converting it into electrical energy in the event of a plant outage, and from that and the energy produced using the energy carrier fractions, the power demand of the fuel production plant is ensured, in particular the operation of the safety equipment of the plant.

10. A system (100) for producing a climate-neutral fuel, **characterized in** comprising:
- a carbon dioxide production first unit (20) for extracting carbon dioxide from the environment and a hydrogen production second unit (30) for producing hydrogen from water,
- one or more hydrocarbon mixture production unit (50), connected to the first and second units (20, 30), for producing a hydrocarbon mixture from carbon dioxide and hydrogen gases by a one or more step chemical and/or electrochemical process,
- a fractionation unit (60) for separating the hydrocarbon mixture into one or more fuel fractions (5a) and one or more energy carrier fractions (5b),
said system (100) further comprises solar panels (10) and one or more thermal power plant (70) for utilizing the one or more energy carrier fractions (5b) and, optionally, a part of the one or more fuel fractions (5a), which solar panels (10) and one or more thermal power plant (70) are connected to said units (20, 30, 40, 50, 60) and which are adapted to generate the energy required to operate said units (20, 30, 40, 50, 60).

11. The system (100) according to claim 10, **characterised in that** it comprises a unit (40) for producing carbon monoxide from carbon dioxide and for producing a synthesis gas from carbon monoxide and hydrogen, which unit (40) is coupled to the units (20, 30), and a conversion unit (50) for producing a hydrocarbon mixture from the synthesis gas, coupled to the reaction unit (40).

12. The system (100) according to claim 10 or 11, **characterized in that** the solar panels (10) are sized to meet the combined energy demand of the units (20, 30, 40, 50, 60).

13. The system (100) according to any one of claims 10 to 12, **characterized in that** the one or more thermal power plants (70) are sized to meet the combined energy demand of the units (20, 30, 40, 50, 60).
